# EUROPEAN PATENT SPECIFICATION

(11) **EP 3 236 913 B1**
(45) Date of publication and mention of the grant of the patent: **01.04.2020**
(21) Application number: 15831051.6
(22) Date of filing: 22.12.2015
(51) Int. Cl.: A61K 8/29, A61K 8/35, A61K 8/37, A61K 8/49, A61Q 17/00, A61Q 17/04, A61K 8/04, A61K 8/06

(54) **COSMETIC SUNSCREEN COMPOSITION**
EMULGATORFREIE KOSMETISCHE SONNENSCHUTZMITTELZUSAMMENSETZUNG
COMPOSITION D'ÉCRAN SOLAIRE COSMÉTIQUE SANS ÉMULSIFIANT

(30) Priority: 22.12.2014 EP 14004350
(43) Date of publication of application: 01.11.2017
(73) Proprietor: Galderma S.A., 6330 Cham (CH)
(72) Inventor: BOHNENBLUST, Katharina, 4622 Egerkingen (CH)
(74) Representative: Nederlandsch Octrooibureau
(86) International application number: PCT/EP2015/002592
(87) International publication number: WO 2016/102064

(56) References cited:
- EP-A1- 2 745 831
- WO-A1-2010/031522
- WO-A2-2013/102248
- US-A1- 2009 202 459
- US-A1- 2013 085 186

## Description

This invention relates to a sun protection composition. More particularly, the invention relates to a composition formulated as a gel-cream having a Sun Protection Factor (SPF) greater than or equal to 25. The invention also relates to a method for manufacturing such a sun protection composition as well as its use, in particular for protecting the skin from UV-A and/or UV-B radiation.

Solar radiation is known to have beneficial effects but especially harmful effects in Man. Solar radiation has in particular an anti-rickets action, by assisting in the synthesis of vitamin D, improves certain dermatoses, has an antidepressant action, and allows the skin to darken. However, overexposure to solar radiation can induce an alteration of the skin.

Solar radiation comprises ultraviolet (UV) radiation which is very powerful. Among ultraviolet radiation, only UV-A and UV-B radiation reach the earth and have effects on the skin. UV-C radiation is stopped by the ozone layer.

UV-A rays, with wavelengths between 320 and 400 nm, cause the skin to darken. They are present all throughout the year and even in cloudy weather. They represent 95% of the UVs that touch the surface of the earth. Contrary to UV-B radiation, they are painless and can penetrate the skin very deeply, to the cells of the dermis. UV-A radiation in particular causes:
- photo-ageing, *i*.*e*. the modification of the orientation of the elastin and collagen fibres, causing a slackening and a loss of firmness of the skin and a premature ageing by the appearance of wrinkles;
- sun allergies or photo-allergies (redness, itching, summer lucitis);
- pigmentary disorders (pregnancy mask, freckles); and
- the development of skin cancers.

As such, during an exposure to natural or artificial rays, it is important to filter the UV-A radiation.

UV-B rays, with wavelengths between 280 and 320 nm are high in energy. UV-B rays are responsible for tanning but also for burns (sunburn) as well as allergic reactions and skin cancers.

In light of the above, it therefore appears essential to protect the skin from both UV-A radiation and from UV-B radiation.

Many sun protection compositions (UV-A and/or UV-B) have been proposed to date. Such compositions comprising several sun filters are in particular described in documents FR2975590, FR2983718, WO2010/031522, US2009/202459, WO2013/102248, US2013/085186 and EP2745831.

There is however a need to develop sun protection compositions that are easy and fast to apply, and which are simple to use. Such sun protection compositions must moreover comply with the regulations in terms of SPF (Sun Protection Factor.

Sun protection compositions that have a high SPF are primarily in the form of creams or lotions stabilized by emulsifiers. However, conventional emulsifiers, as they are amphiphilic molecules, tend to interact with the physiological structure of the stratum corneum and could therefore negatively influence sensitive skin. In addition, the presence of some conventional emulsifiers could also lead to phototoxic reactions.

There remains therefore a need to have a wide range of sun protection compositions, which are easy to apply and stable and which are suitable for sensitive or dry skin because of not containing conventional emulsifiers.

According to a first aspect of the present invention, there is provided an Oil in Water (O/W) emulsion comprising, in a physiologically acceptable medium:
- at least one UV filter;
- an emulsion stabilizing and viscosity controlling agent;
- a gelling agent;
wherein it further comprises at least:
- two chelating agents selected from alpha-cyclodextrin (Cavamax™ W6 Food) and trisodium Ethylenediamine Disuccinate (Natrlquest™ E30); and
- a surfactant.

Surprisingly, the Applicant has revealed that it was possible to develop sun protection compositions with high sun protection, i.e. that have an SPF (Sun Protection Factor greater than or equal to 25, preferably greater than or equal to 30 and that are stable without containing any conventional emulsifier. In addition, the combination of three excipients helped to obtain a very smooth formulation without showing gritty structures after storage at elevated temperature. These three excipients, which are two chelating agents and a surfactant need to be used in combination to obtain this result. These excipients are preferably Cavamax W6, Carbopol Ultrez 20, Natrlquest E 30.

According to a further aspect of the invention , there is provided a method of manufacturing a composition according to the invention, wherein it comprises at least the steps of:
- preparing the oily phase by weighing, melting and/or heating of a first phase comprising at least one UV-filter;
- preparing the aqueous phase by weighing, mixing and homogenising of a second phase comprising at least one emulsion stabilizing and viscosity controlling agent, a gelling agent, chelating agents and a surfactant;

- heating and emulsifying the first phase in the second phase and homogenising;
- recovering a composition according to the invention.

Finally, the invention has for last object the use of a composition according to the invention, for protecting the skin against UV-A and/or UV-B radiation.

Other features and embodiments will be apparent from the following non-limitative description of the invention.

The sun protection composition according to the invention is adapted to a topical application on the skin and therefore contains a physiologically acceptable medium, *i*.*e*. compatible with the skin. The compositions according to the invention comprise a physiologically acceptable medium or at least one pharmaceutically acceptable excipient, chosen according to the pharmaceutical or dermatological form desired.

The sun protection composition according to the invention is a gel-cream or lotion-formulation which consists of an emulsion obtained by the dispersion of an oily phase in an aqueous phase (O/W).

The composition is moreover in a form that is adapted to topical application on the skin.

More preferably, the sun protection composition according to the invention is a gel-cream.

The sun protection composition according to the invention comprises at least one UV filter.

By way of a non-restricted example of a UV filter that can be used, the following can be mentioned:
- derivatives of 1,3,5-triazine-2,4,6-triamine, such as Diethylamino Hydroxybenzoyl Hexyl Benzoate (Uvinul™ A+), Ethyl Hexyl Triazone (Uvinul™ T 150), Bis-ethyl Hexyloxyphenol Methoxyphenyl Triazine (Tinosorb™ S), Octocrylene (Eusolex® OCR), Dimethicodiethyl-benzalmalonate (Parsol® SLX), Di-ethylhexyl-butamido Triazone (Uvasorb® HEB);
- esters of cinnamic acid, such as isoamyl-p-methoxycinnamate or 2-ethylhexyl-p-methoxycinnamate (INCI: Ethyl Hexyl Methoxycinnamate), in particular marketed under the name Eusolex® 9020);
- a mixture of Titanium Dioxide, C12-C15 Alkyl Benzoate, Polyglyceryl-2 Dipolyhydroxystearate, Silica, Dimethicone (Parsol™ TX50 AB);
- derivatives of phenylbenzimidazole, such as Phenylbenzimidazole Sulfonic Acid, (Eusolex® 232);
- derivatives of benzophenone, such as Benzophenone-3 (Tinosorb™ B3), Benzophenone-4 (Uvinul™ MS 40) or Benzophenone-5; and
- derivatives of p-aminobenzoic acid, such as the ethoxylated derivative PEG 25 - PABA (Uvinul™ P25), Methylene Bis-benzotriazolyl Tetramethylbutylphenol (Tinosorb™ M).

More preferably, the composition according to the invention comprises at least one UV filter chosen from among Bis-Ethylhexyloxyphenol Methoxyphenyl Triazine (BEMT) (Tinosorb™ S), Ethylhexyl Methoxycinnamate, Diethylamino Hydroxybenzoyl Hexyl Benzoate (Uvinul™ A+), Ethyl Hexyl Triazone (Uvinul™ T 150), Methylene Bis-benzotriazolyl Tetramethylbutylphenol (Tinosorb™ M) or a mixture of Titanium Dioxide, C12-C15 Alkyl Benzoate, Polyglyceryl-2 Dipolyhydroxystearate, Silica, Dimethicone (Parsol™ TX50 AB).

Advantageously, the composition according to the invention comprises at least two UV filters.

More preferably, the composition according to the invention comprises at least two UV filters chosen from among Bis-Ethylhexyloxyphenol Methoxyphenyl Triazine (BEMT) (Tinosorb™ S), Ethylhexyl Methoxycinnamate, Diethylamino Hydroxybenzoyl Hexyl Benzoate (Uvinul™ A+), Ethyl Hexyl Triazone (Uvinul™ T 150), Methylene Bis-benzotriazolyl Tetramethylbutylphenol (Tinosorb™ M) and/or a mixture of Titanium Dioxide, C12-C15 Alkyl Benzoate, Polyglyceryl-2 Dipolyhydroxystearate, Silica, Dimethicone (Parsol™ TX50 AB).

More advantageously, the composition according to the invention comprises at least three UV filters.

More preferably, the three UV filters are chosen among Bis-Ethylhexyloxyphenol Methoxyphenyl Triazine (BEMT) (Tinosorb™ S), Ethylhexyl Methoxycinnamate, Diethylamino Hydroxybenzoyl Hexyl Benzoate (Uvinul™ A+), Ethyl Hexyl Triazone (Uvinul™ T 150), Methylene Bis-benzotriazolyl Tetramethylbutylphenol (Tinosorb™ M) or a mixture of Titanium Dioxide, C12-C15 Alkyl Benzoate, Polyglyceryl-2 Dipolyhydroxystearate, Silica, Dimethicone (Parsol™ TX50 AB).

According to an advantageous embodiment of the invention the composition comprises one or several UV filters such as hereinabove, at concentrations that allow for the obtaining of a high SPF (Sun Protection Factor, i.e. of at least 25, preferably of at least 30. More preferably, the composition having a SPF of at least 25 (and presenting an UVA-PF of 1/3 of the SPF) comprises a mixture of UV filters:
- Bis-Ethylhexyloxyphenol Methoxyphenyl Triazine (BEMT) (Tinosorb™ S),
- Ethylhexyl Methoxycinnamate and
- Diethylamino Hydroxybenzoyl Hexyl Benzoate (Uvinul™ A+).

Even more preferably, the composition having a SPF of at least 25 comprises a mixture of the following UV filters:
- between 2.0% and 7.0% of Bis-Ethylhexyloxyphenol Methoxyphenyl Triazine (BEMT) (Tinosorb™ S), preferably between 3% and 5%;
- between 5.0% and 15% of Ethylhexyl Methoxycinnamate, preferably between 7% and 12%; and
- between 2.0% and 9.0% of Diethylamino Hydroxybenzoyl Hexyl Benzoate (Uvinul™ A+), preferably between 3% and 5%.

For example, a composition containing the following UV filters:
- 2.5% of Bis-Ethylhexyloxyphenol Methoxyphenyl Triazine (BEMT) (Tinosorb™ S);
- 10% of Ethylhexyl Methoxycinnamate; and
- 4.0% of Diethylamino Hydroxybenzoyl Hexyl Benzoate (Uvinul™ A+)
has a SPF of 31.7 and an UVA-PF of 8.5.

In the same way, a composition containing the following UV filters:
- 5% of Bis-Ethylhexyloxyphenol Methoxyphenyl Triazine (BEMT) (Tinosorb™ S);
- 7% of Ethylhexyl Methoxycinnamate; and
- 4.0% of Diethylamino Hydroxybenzoyl Hexyl Benzoate (Uvinul™ A+)
has a SPF of 32.6 and an UVA-PF of 11.4.

In the same way, a composition containing the following UV filters:
- 2% of Bis-Ethylhexyloxyphenol Methoxyphenyl Triazine (BEMT) (Tinosorb™ S) ;
- 8% of Ethylhexyl Methoxycinnamate; and
- 3.5% of Diethylamino Hydroxybenzoyl Hexyl Benzoate (Uvinul™ A+)

has a SPF of 25.7 and an UVA-PF of 8.9.According to another advantageous embodiment of the invention the composition comprises one or several UV filters such as hereinabove, at concentrations that allow for the obtaining of a high SPF (Sun Protection Factor, i.e. of at least 45, preferably of at least 50. More preferably, the composition having a SPF of at least 45 comprises a mixture of the following UV filters:
- Bis-Ethylhexyloxyphenol Methoxyphenyl Triazine (BEMT) (Tinosorb™ S),
- Ethylhexyl Methoxycinnamate,
- Diethylamino Hydroxybenzoyl Hexyl Benzoate (Uvinul™ A+),
- Ethyl Hexyl Triazone (Uvinul™ T 150),
- Methylene Bis-benzotriazolyl Tetramethylbutylphenol (Tinosorb™ M)
- A mixture of Titanium Dioxide, C12-C15 Alkyl Benzoate, Polyglyceryl-2 Dipolyhydroxystearate, Silica, Dimethicone (Parsol™ TX50 AB).

Even more preferably, the composition having a SPF of at least 45 comprises a mixture of the following UV filters:
- between 2.0% and 7.0% of Bis-Ethylhexyloxyphenol Methoxyphenyl Triazine (BEMT) (Tinosorb™ S),
- between 5.0% and 15.0% of Ethylhexyl Methoxycinnamate,
- between 5.0% and 10.0% of Diethylamino Hydroxybenzoyl Hexyl Benzoate (Uvinul™ A+),
- between 0.05% and 2.0% of Ethyl Hexyl Triazone (Uvinul™ T 150),
- between 1.0% and 3.0% of Methylene Bis-benzotriazolyl Tetramethylbutylphenol (Tinosorb™ M),
- between 5.0% and 15.0% of a mixture of Titanium Dioxide, C12-C15 Alkyl Benzoate, Polyglyceryl-2 Dipolyhydroxystearate, Silica, Dimethicone (Parsol™ TX50 AB).

For example, a composition containing the following UV filters:
- 4.0% of Bis-Ethylhexyloxyphenol Methoxyphenyl Triazine (BEMT) (Tinosorb™ S),
- 7.0% of Ethylhexyl Methoxycinnamate,
- 7.5% of Diethylamino Hydroxybenzoyl Hexyl Benzoate (Uvinul™ A+),
- 1.0% of Ethyl Hexyl Triazone (Uvinul™ T 150),
- 2.0% of Methylene Bis-benzotriazolyl Tetramethylbutylphenol (Tinosorb™ M)
- 20.0% of a mixture of Titanium Dioxide, C12-C15 Alkyl Benzoate, Polyglyceryl-2 Dipolyhydroxystearate, Silica, Dimethicone (Parsol™ TX50 AB).
has a calculated SPF of 59.8 and a calculated UVA-PF of 21.6.

For example, a composition containing the following UV filters:
- 3.0% of Bis-Ethylhexyloxyphenol Methoxyphenyl Triazine (BEMT) (Tinosorb™ S),
- 7.0% of Ethylhexyl Methoxycinnamate,
- 5.0% of Diethylamino Hydroxybenzoyl Hexyl Benzoate (Uvinul™ A+),
- 1.0% of Ethyl Hexyl Triazone (Uvinul™ T 150),
- 2.0% of Methylene Bis-benzotriazolyl Tetramethylbutylphenol (Tinosorb™ M)
- 10.0% of a mixture of Titanium Dioxide, C12-C15 Alkyl Benzoate, Polyglyceryl-2 Dipolyhydroxystearate, Silica, Dimethicone (Parsol™ TX50 AB).
has a calculated SPF of 46.4 and a calculated UVA-PF of 11.1.

For example, a composition containing the following UV filters:
- 5.0% of Bis-Ethylhexyloxyphenol Methoxyphenyl Triazine (BEMT) (Tinosorb™ S),
- 8.0% of Ethylhexyl Methoxycinnamate,
- 5.0% of Diethylamino Hydroxybenzoyl Hexyl Benzoate (Uvinul™ A+),
- 0.5% of Ethyl Hexyl Triazone (Uvinul™ T 150),
- 3.0% of Methylene Bis-benzotriazolyl Tetramethylbutylphenol (Tinosorb™ M)
- 12.0% of a mixture of Titanium Dioxide, C12-C15 Alkyl Benzoate, Polyglyceryl-2 Dipolyhydroxystearate, Silica, Dimethicone (Parsol™ TX50 AB).
has a calculated SPF of 57.2 and a calculated UVA-PF of 17.0.

The composition according to the invention comprises an emulsion stabilizing and viscosity controlling agent. An emulsion stabilizing and viscosity controlling agent is an agent creating a kind of network where droplets are fixed.

An emulsion stabilizing and viscosity controlling agent is distinct from a conventional emulsifier as it is not HLB (Hydrophilic/Lipophilic Balance) dependent and provides hydrophobic interactions providing a (microgel)network which captures/entraps the oil droplets whereas a conventional emulsifier is HLB dependent and strongly reduces the interfacial oil-water tension, sometimes providing a lamellar phase.

The emulsion stabilizing and viscosity controlling agent encompassed in the invention has not the role of reducing the interfacial tension. Thus, the formulations of invention are devoid of conventional emulsifier.

Preferably, the emulsion stabilizing agent is a polymer.

By way of a non-restricted example of emulsion stabilizing and viscosity controlling agent that can be used, the following can be mentioned:
- acrylate and acrylamide polymers such as Acrylates/C10-30 Alkyl Acrylate Crosspolymer (Pemulen™ TR2).

The composition according to the invention also comprises a gelling agent, which is preferably the gelling agent xanthan gum, also marketed in particular under the name Xantural™ 75.

Surprisingly, the Applicant was able to reveal that, in order to obtain a stable composition having good sensory and macroscopic parameters (in term of, for example colour, change in colour, odour, shininess, superficial properties, consistency, oily and/or aqueous deposit, granularity, formation of a protuberance, ...) the composition according to the invention should necessarily comprise two chelating agents and a surfactant, but no emulsifying agent such as, for example, Lecithin, Cetyl Phosphate, , Montanov™ 68 (Cetearyl Alcohol and Cetearyl Glucoside).

By way of a non-restricted example of a chelating agents that can be used, alpha-cyclodextrin (Cavamax™ W6 Food) and trisodium Ethylenediamine Disuccinate (Natrlquest™ E30) can be mentioned. More preferably, the chelating agent are alpha-cyclodextrin (Cavamax™ W6 Food) and Trisodium Ethylenediamine Disuccinate (Natrlquest™ E30). These two chelating agents are preferably used because they enable droplet stabilisation.

The composition according to the invention further comprises a surfactant which makes it possible to favour an even distribution of the product when it is used. The surfactant also prevents the "cottage-cheese-effect" at long-term storage. Indeed, the "cottage-cheese-effect" is not acceptable for customers from a cosmetic point of view.

By way of a non-restricted example of surfactant that can be used, Lauryl Glucoside (Plantacare™ 1200 UP or Carbopol Ultrez 20) can be mentioned.

Advantageously, the composition according to the invention does not contain any conventional emulsifying agent, *i*.*e*. no compound that favours the formation of intimate mixtures between immiscible liquids such as oil and water. Such conventional emulsifying agents are for example chosen from among Lecithin, Cetyl Phosphate, , Montanov™ 68

### (Cetearyl Alcohol and Cetearyl Glucoside),.

Advantageously, the composition according to the invention further comprises one or several components, taken individually or as a mixture, such as:
- an emmolient, such as:
   ∘ benzoate esters such as Dipropylene Glycol Dibenzoate (Finsolv® PG-22), Ethylhexyl Benzoate (Finsolv® EB) or C12-15 Alkyl Benzoate;
   ∘ Dicaprylyl Carbonate (Cetiol® CC) and
   ∘ Dibutyl Adipate.
- pH regulators such as Triethanolamine (Trolamine™ EP) and Sodium Hydroxide solution;
- a film-forming agent, such as acetyl tributyl citrate, Antaron™ polymers such as the VP/Hexadecene copolymer (Antaron™ (Ganex) V-216 Polymer), the VP/Eicosene copolymer (Antaron™ (Ganex) V-220/220F polymer and Triacontanyl PVP (Antaron™ (Ganex) WP-660 polymer); waxes such as in particular Cera Alba, Cera candelilla or Cera carnauba; cellulose and derivatives thereof (acetates, nitrates, etc.); and chitosan and derivatives thereof;
- skin-conditioning agents such as Dimethicone 5 CS;
- an antioxidant such as dl-alpha-Tocopherol;
- a co-solvent such as ethanol; and
- purified water.

According to a preferred embodiment of the invention, the composition according to the invention comprises at least:
- one or more UV filters which are chosen from among Bis-Ethylhexyloxyphenol Methoxyphenyl Triazine (BEMT) (Tinosorb™ S), Ethylhexyl Methoxycinnamate or Diethylamino Hydroxybenzoyl Hexyl Benzoate (Uvinul™ A+), taken individually or as a mixture;
- one emulsion stabilizing and viscosity agent which is Acrylates/C10-30 Alkyl Acrylate Crosspolymer (Pemulen™ TR2);
- one gelling agent which is xanthan gum, (Xantural™ 75)
- two chelating agents which are alpha-cyclodextrin (Cavamax™ W6 Food) and trisodium Ethylenediamine Disuccinate (Natrlquest™ E30); and
- one surfactant which is Lauryl Glucoside (Plantacare™ 1200 UP or Carbopol Ultrez 20).

More preferably, the composition further comprises:
- Purified Water;
- Triethanolamine (Trolamine™ EP);
- VP/Eicosene Copolymer (Antaron™ V-220 F);
- Dimethicone;
- Dibutyl Adipate
- C12-15 Alkyl Benzoate
- dl-alpha-Tocopherol; and
- ethanol.

Even more preferably, the composition according to the invention comprises the following constituents in the following proportions, expressed as a percentage in weight of the total weight of the composition:
- 30% to 70% purified water;
- 9.0% to 31% of one or more UV filters which are chosen from among Bis-Ethylhexyloxyphenol Methoxyphenyl Triazine (BEMT) (Tinosorb™ S), Ethylhexyl Methoxycinnamate, Diethylamino Hydroxybenzoyl Hexyl Benzoate (Uvinul™ A+), Ethyl Hexyl Triazone (Uvinul™ T 150), Methylene Bis-benzotriazolyl Tetramethylbutylphenol (Tinosorb™ M) or a mixture of Titanium Dioxide, C12-C15 Alkyl Benzoate, Polyglyceryl-2 Dipolyhydroxystearate, Silica, Dimethicone (Parsol™ TX50 AB) taken individually or as a mixture;
- 0.05% to 0.3% of an emulsion stabilizing and viscosity agent which is Acrylates/C10-30 Alkyl Acrylate Crosspolymer (Pemulen™ TR2);
- 0.05% to 0.3% of a gelling agent which is xanthan gum, (Xantural™ 75)
- 0.05% to 3% of a chelating agents which are alpha-cyclodextrin (Cavamax™ W6 Food) and Trisodium Ethylenediamine Disuccinate (Natrlquest™ E30); and
- 0.01% to 0.3% of a surfactant which is Lauryl Glucoside (Plantacare™ 1200 UP or Carbopol Ultrez 20).

In a preferred embodiment, the composition comprises:
- 4.0% to 5.0% of Bis-Ethylhexyloxyphenol Methoxyphenyl Triazine (BEMT) (Tinosorb™ S);
- 8.0% to 10.0% of Ethylhexyl Methoxycinnamate; and
- 5.0% to 6.0% of Diethylamino Hydroxybenzoyl Hexyl Benzoate (Uvinul™ A+).

In a second preferred embodiment, the composition comprises:
- 4.0% to 5.0% of Bis-Ethylhexyloxyphenol Methoxyphenyl Triazine (BEMT) (Tinosorb™ S),
- 8.0 to 10.0% of Ethylhexyl Methoxycinnamate,
- 6.0% to 7.5% of Diethylamino Hydroxybenzoyl Hexyl Benzoate (Uvinul™ A+),
- 0.5% to 1.0% of Ethyl Hexyl Triazone (Uvinul™ T 150),
- 2.0% to 3.0% of Methylene Bis-benzotriazolyl Tetramethylbutylphenol (Tinosorb™ M)
- 10.0% to 20.0% of a mixture of Titanium Dioxide, C12-C15 Alkyl Benzoate, Polyglyceryl-2 Dipolyhydroxystearate, Silica, Dimethicone (Parsol™ TX50 AB).

Preferably, the composition according to the invention does not contain any conventional emulsifier.

The invention also relates to a method of manufacturing a composition according to the invention, wherein it comprises at least the steps of:
- preparing the oily phase by weighing, melting and/or heating of a first phase comprising at least one UV-filter, preferably chosen among Bis-Ethylhexyloxyphenol Methoxyphenyl Triazine (BEMT) (Tinosorb™ S), ethylhexyl Methoxycinnamate diethylamino Hydroxybenzoyl Hexyl Benzoate (Uvinul™ A+), Ethyl Hexyl Triazone (Uvinul™ T 150);
- preparing the aqueous phase by weighing, mixing and homogenising of a second phase comprising at least one emulsion stabilizing and viscosity controlling agent, preferably an acrylate and acrylamide polymer such as Acrylates/C10-30 Alkyl Acrylate Crosspolymer (Pemulen™ TR2), a gelling agent which is preferably the gelling agent xanthan gum, chelating agents which are preferably alpha-cyclodextrin (Cavamax™ W6 Food) and Trisodium Ethylenediamine Disuccinate (Natrlquest™ E30) and a surfactant which is preferably Carbopol Ultrez 20 (Lauryl Glucoside, Acrylates/C10-30 Alkyl Acrylate Crosspolymer) or Plantacare™ 1200 UP (Lauryl Glucoside);
- heating and emulsifying the first phase in the second phase and homogenising;
- recovering a composition according to the invention.

More preferably, the first phase further comprises:
- C12-15 Alkyl Benzoate,
- Dibutyl Adipate,
- VP/Eicosene Copolymer (Antaron™ V-220 F) and/or
- Dimethicone,
and the second phase further comprises triethanolamine and purified water.

In a particular embodiment, a third phase comprising dl-alpha-Tocopherol and Ethanol 96% EP is added before the step of the recovering of the composition according to the invention.

Advantageously, for the manufacture of composition having an SPF greater than 45, the method according to the invention further contains the adding of a fourth phase comprising Methylene Bis-benzotriazolyl Tetramethylbutylphenol (Tinosorb™ M) and of a fifth phase comprising a mixture of Titanium Dioxide, C12-C15 Alkyl Benzoate, Polyglyceryl-2 Dipolyhydroxystearate, Silica, Dimethicone (Parsol™ TX50 AB), before the step of the recovering of the composition according to the invention.

Finally, the invention relates to the use of a composition according to the invention, for protecting the skin against UV-A and/or UV-B radiation.

More preferably, the compositions are particularly effective in protecting human skin against the harmful effects of UV-A and/or UV-B radiation which include but are not limited to photo-ageing, burns, sun allergies or photo-allergies, pigmentary disorders and the development of skin cancers.

As such, this invention also relates to a method for protecting human skin against the harmful effects of UV-A and/or UV-B radiation, by application of a composition according to the invention.

This invention shall now be illustrated by means of the following examples. In these examples, the quantities are expressed as a percentage by weight.

### Example 1: Sun protection compositions:

The Applicant manufactured and analysed ten different sun protection compositions numbered 41, 42, 43, 44, 45, 46, 47, 48, 99 and 166.

Table 1 herein below provides the qualitative and quantitative content of the compositions tested:

**Table 1:**

| **Compositions** | | **41** | **42** | **43** | **44** | **45** |
|---|---|---|---|---|---|---|
| **Phases** | **Constituents** | **%** | **%** | **%** | **%** | **%** |
| **A** | Purified water (qsp) | from 55.000% to 65.000% | | | | |
| | Pemulen TR2 (Acrylates/C10-30 Alkyl Acrylate Crosspolymer) | from 0.010% to 0.200% | | | | |
| | Xantural™ 75 (Xanthan Gum) | from 0.05% to 0.300% | | | | |
| | Carbopol Ultrez 20 (Lauryl Glucoside, Acrylates/C10-30 Alkyl Acrylate Crosspolymer) | from 0.05% to 0.200% | | | | |
| | Natrlquest™ E30 (Trisodium Ethylenediamine Disuccinate) | from 0.050% to 0.200% | from 0.050% to 0.200% | - | - | from 0.050% to 0.200% |
| | Cavamax™ W6 Food (alpha-cyclodextrine) | from 0.500% to 2.000% | | | | - |
| | Trolamine™ EP (Triethanolamine) | from 0.050% to 1.000% | | | | |
| **B** | C12-15 Alkyl Benzoate | from 1.500% to 4.500% | - | from 1.500% to 4.500% | - | from 1.500% to 4.500% |
| | Dibutyl Adipate | from 1.000% to 5.000% | - | from 1.000% to 5.000% | - | from 1.000% to 5.000% |
| | Antaron™ V-220 F (VP /Eicosene Copolymer) | from 0.100% to 3.000% | | | | |
| | Tinosorb™ S (Bis-Ethylhexyloxypheno l Methoxyphenyl Triazine (BEMT) | from 2.000% to 5.000% | | | | |
| | Ethylhexyl Methoxycinnamate | from 7.000% to 10.000% | | | | |
| | Uvinul™ A+ (Diethylamino Hydroxybenzoyl Hexyl Benzoate) | from 3.500% to 5.000% | | | | |
| | Dimethicone 5 CS | from 0.100% to 5.000% | | | | |
| **C** | dl-alpha-Tocopherol# | from 0.100% to 2.000% | | | | |
| | Ethanol 96 % EP | from 6.000% to 13.000% | | | | |
| | | | | | | |

| **Compositions** | | **46** | **47** | **48** | **99** | **166** |
|---|---|---|---|---|---|---|
| **Phases** | **Constituents** | **%** | **%** | **%** | **%** | **%** |
| **A** | Purified water (qsp) | from 35.000% to 70.000% | | | | |
| | Pemulen TR2 (Acrylates/C10-30 Alkyl Acrylate Crosspolymer) | from 0.010% to 0.200% | | | | |
| | Xantural™ 75 (Xanthan Gum) | from 0.050% to 0.300% | | | | |
| | Carbopol Ultrez 20 (Lauryl Glucoside, Acrylates/C10-30 Alkyl Acrylate Crosspolymer) | from 0.050% to 0.200% | | | | - |
| | Natrlquest™ E30 (Trisodium Ethylenediamine Disuccinate) | from 0.050% to 0.200% | - | - | from 0.050% to 0.200% | from 0.050% to 0.200% |
| | Cavamax™ W6 Food (alpha-cyclodextrine) | - | - | - | from 0.500% to 2.000% | from 0.500% to 2.000% |
| | Plantacare™ 1200 UP (Lauryl Glucoside) | - | - | - | - | from 0.010% to 0.200% |
| | Trolamine™ EP (Triethanolamine) | from 0.050% to 1.000% | | | | |
| **B** | C12-15 Alkyl Benzoate | - | from 1.500% to 4.500% | - | from 1.500% to 4.500% | from 1.500% to 4.500% |
| | Dibutyl Adipate | - | from 1.000% to 5.000% | - | from 1.000% to 5.000% | from 1.000% to 5.000% |
| | Antaron™ V-220 F (VP/Eicosene Copolymer) | from 0.100% to 3.000% | | | | |
| | Tinosorb™ S (Bis-Ethylhexyloxypheno l Methoxyphenyl Triazine (BEMT) | from 2.000% to 5.000% | | | | |
| | Octinoxate USP (Ethylhexyl Methoxycinnamate) | from 7.000% to 10.000% | | | | |
| | Ethylhexyl Triazone | - | - | - | - | from 0.100% to 2.000% |
| | Uvinul™ A+ (Diethylamino Hydroxybenzoyl Hexyl Benzoate) | from 3.500% to 8.000% | | | | |
| | Dimethicone 5 CS | from 0.100% to 5.000% | | | | |
| **C** | d1-alpha-Tocopherol# | from 0.100% to 2.000% | | | | |
| | Ethanol 96 % EP | from 6.000% to 13.000% | | | | |
| **D** | Tinosorb™ M (Methylene Bis-Benzotriazolyl Tetramethylbutylph enol (and) Aqua (and) Decyl Glucoside (and) Propylene Glycol (and) Xanthan Gum) | - | - | - | - | from 0.100% to 3.000% |
| **E** | Parsol™ TX50 AB (Titanium Dioxide, C12-C15 Alkyl Benzoate, Polyglyceryl-2 Dipolyhydroxystear ate, Silica, Dimethicone) | - | - | - | - | from 7.000% to 10.000% |

Preferably, the composition according to the invention is an O/W-Emulsion (called "Gel"), where the oil droplets are only stabilized by means of Pemulen TR2, which is a emulsion stabilizing and viscosity controlling agent (Carbomer Polymer) and Xanthan Gum, which is a gelling agent. It does not contain any conventional emulsifier.

In the first development cycle of the project, the emulsion turned out to be stable, but for both SPFs, 30 and 50+, a phenomenon occurred over storage, which can be described as "cottage cheese effect".

Samples at 40°C and later at room temperature developed an inhomogeneous structure which can be compared to the appearance of "cottage cheese". It appears that this is a well-known problem when using Pemulen TR2.

A range of additional experiments were conducted to evaluate the influence of different stabilizing excipients in order to overcome this instability issue.

Finally, a fixed combination was found that was particularly able to provide a nice, stable and smooth texture of the composition. This composition advantageously contains:
- 0.1% to 0.15% Pemulen TR2;
- 0.1% to 0.2% Xanthan Gum; together with
- 0.050% to 0.200% Natrlquest E30
- 0.500% to 2.000% Cavamax W6 Food
- 0.05% to 0.200% Carbopol Ultrez 20 (contains Plantacare 1200 UP) or 0.010% to 0.200%% Plantacare 1200 UP.

The table above shows different combinations which were evaluated. By leaving out Natrlquest E30 or Cavamax W6 Food or both together, the "cottage cheese effect" was still there. Thus, compositions 43 to 48 are not completely satisfactory.

There are also additional experiments (not in the table) showing that Carbopol Ultrez 20 (which contains Plantacare 1200) or Plantacare 1200 (surfactant), is necessary and that only the combination of these excipients led to the desired effect (Cavamax™ W6 Food, Natrlquest™ E30, Carbopol Ultrez 20 and/or Plantacare 1200).

Preferably, the composition should contain:
- Cavamax™ W6 Food;
- Natrlquest™ E30; and
- Carbopol Ultrez 20 or Plantacare 1200.

The preferred formula for SPF 30 (composition 99) and also the one for SPF 50 (composition 166) preferably contains this specific combination. The SPF 30 composition 99 contains Carbopol Ultrez, which contains Plantacare 1200 UP, whereas for composition 166 (SPF 50), Plantacare 1200 UP was directly added.

Other compositions numbered 49 to 52 were also manufactured by the Applicant. These compositions did not contain Carbopol Ultrez 20 but an increased concentration of Xanthan Gum instead (0,25% for compositions 49 and 50 and 0,3% for compositions 51 and 52). They did not show a cottage cheese effect, but showed increase of droplet size and oil separation. Thus, these composition were not useful.

A composition numbered 100 was also manufactured by the Applicant containing a different UV-filter package compared to composition 99. This composition 100 did not fulfil the SPF requirements and was therefore also not useful.

Compositions numbered 101 and 102 were also manufactured by the Applicant. They respectively mirror compositions 99 and 100 but with 0,3% Xanthan Gum instead of Carbopol. These compositions were not stable over storage. Therefore, these compositions 101 and 102 are also not relevant and not useful.

### Example 2: Method for manufacturing sun protection compositions 41 to 48 according to example 1:

The compositions 41 to 48 were prepared according to the following steps:
1. weighing of the various constituents of phase B in a glass beaker;
2. melting of the various constituents of phase B under agitation using a magnetic stirrer hot plate;
3. weighing of the various constituents of phase A in a glass beaker;
4. mixing and homogenising of the various constituents of phase A;
5. adjusting the pH-value;
6. compensation with water and homogenization;
7. heating of phase A to 80°C;
8. emulsifying of phase B in phase A by homogenising;
9. cooling the gel under stirring with the anchor stirrer to room temperature; and
10. adding phase C and homogenising of the whole.

### Example 3: Method for manufacturing sun protection compositions 99 according to example 1:

The composition 99 was prepared according to the following steps:
1. weighing of the various constituents of phase B in a glass beaker;
2. melting of the various constituents of phase B under agitation using a magnetic stirrer hot plate;
3. weighing of the various constituents of phase A in a glass beaker;
4. mixing and homogenising of the various constituents of phase A;
5. adjusting the pH-value on 4;
6. compensation with water and homogenization;
7. heating of phase A to 80°C;
8. emulsifying of phase B in phase A by homogenising;
9. cooling the gel under stirring with the anchor stirrer to room temperature; and
10. adding phase C and homogenising of the whole.

### Example 4: Method for manufacturing sun protection compositions 166 according to example 1:

The composition 166 was prepared according to the following steps:
1. weighing of the various constituents of phase B in a glass beaker;
2. melting of the various constituents of phase B under agitation using a magnetic stirrer hot plate until a temperature of approximatively 82°C;
3. weighing of the various constituents of phase A, except for Trolamine™ EP, in a glass beaker;
4. mixing and homogenising of the various constituents of phase A except for Trolamine™ EP;
5. weighing of Trolamine™ EP and homogenising in the phase A;
7. heating of phase A with to approximatively 82°C;
8. emulsifying of phase B in phase A by homogenising;
9. cooling the gel under stirring with the anchor stirrer to room temperature;
10. adding phase C and homogenising of the whole;
11. adding phase D and homogenising of the; and
12. adding phase D and homogenising of the whole.

### Example 5: Galenic evaluations of the composition 41:

The quality and the stability of the composition 41 were evaluated under the microscope and by a temperature test. The results of the evaluation under the microscope of the composition 41 (Axioskop, Zeiss) are provided in table 2 hereinbelow:

**Table 2:**

| **Time (weeks)** | **Evaluation with polarizer** | **Evaluation on brightfield** |
|---|---|---|
| T0 (After manufacture) | Absence of crystals | Homogeneous composition, gel structures No presence of droplets |
| T+4 weeks at room temperature (RT) | Absence of crystals | Homogeneous composition, gel structures No presence of droplets |
| T+8 weeks at RT | Absence of crystals | Homogeneous composition, gel structures No presence of droplets |
| T+8 weeks at +4°C | Absence of crystals | Homogeneous composition, gel structures No presence of droplets |
| T+12 weeks at RT | Absence of crystals | Homogeneous composition, gel structures No presence of droplets |
| T+12 weeks at +4°C | Absence of crystals | Homogeneous composition, gel structures No presence of droplets |
| T+26 weeks at RT | Absence of crystals | Homogeneous composition, gel structures with a size of less than 50µm Droplets with a size of less than 10µm |
| T+26 weeks at +4°C | Absence of crystals | Homogeneous composition, gel structures with a size of less than 50µm Droplets with a size of less than 10µm |

The results of the temperature tests of the composition 41 are provided in table 3 herein below. During the temperature tests, sensory and macroscopic parameters were verified, such as in particular the colour, the change in colour, the odour, the shininess, the superficial properties, the consistency, the oily and/or aqueous deposit, the granularity, formation of a protuberance.

**Table 3:**

| **Time (weeks)** | **Macroscopic and sensory properties of the composition** |
|---|---|
| T0 (After manufacture) | Ethanol odour, yellow, shiny, smooth composition, lightly form stable, not fluid and minimal gritty (due to small air bubbles) |
| T+2 weeks stress test (RT-60°C-RT) | Minimal oil deposits on the surface |
| T+4 weeks at +4°C | Identical to T0 |
| T+4 weeks at RT | Identical to T0 |
| T+4 weeks at +30°C | Identical to T0 |
| T+4 weeks at +40°C | Identical to T0 |
| T+8 weeks at +4°C | Identical to T0 |
| T+8 weeks at RT | 1 black dot (approx 1x1mm) at the lower snap-cap edge, otherwise identical to T0 |
| T+8 weeks at +30°C | Identical to T0 |
| T+8 weeks at +40°C | Identical to T0 |
| T+12 weeks at +4°C | Identical to T0 |
| T+12 weeks at RT | Identical to T0 |
| T+12 weeks at +30°C | Identical to T0 |
| T+12 weeks at +40°C | Identical to T0 |
| T+26 weeks at +4°C | Identical to T0 |
| T+26 weeks at RT | Identical to T0 |
| T+26 weeks at +30°C | Identical to T0 |
| T+26 weeks at +40°C | minimal deposition on the surface |

As indicated at example 1, no "cottage cheese effect" was observed for composition 41.

### Example 6: Galenic evaluation of the composition 42 :

The quality and the stability of the composition 42 were evaluated under the microscope and by a temperature test.

The results of the evaluation under the microscope of the composition 42 (Axioskop, Zeiss) are provided in table 4 hereinbelow:

**Table 4:**

| **Time (weeks)** | **Evaluation with polarizer** | **Evaluation on brightfield** |
|---|---|---|
| T0 (After manufacture) | Absence of crystals | Homogeneous composition, gel structure No presence of droplets |
| T+4 weeks at room temperature (RT) | Absence of crystals | Homogeneous composition, gel structure No presence of droplets |
| T+8 weeks at RT | Absence of crystals | Homogeneous composition, gel structure No presence of droplets |
| T+8 weeks at +4°C | Absence of crystals | Homogeneous composition, gel structure No presence of droplets |
| T+12 weeks at RT | Absence of crystals | Homogeneous composition, gel structure No presence of droplets |
| T+12 weeks at +4°C | Absence of crystals | Homogeneous composition, gel structure No presence of droplets |
| T+26 weeks at RT | Absence of crystals | Homogeneous composition, gel structure with a size of less than 50µm Droplets with a size of less than 15µm |
| T+26 weeks at +4°C | Absence of crystals | Homogeneous composition, gel structure with a size of less than 50µm Droplets with a size of less than 10µm |

The results of the temperature tests of the composition 42 are provided in table 5 herein below. During the temperature tests, sensory and macroscopic parameters were verified, such as in particular the colour, the change in colour, the odour, the shininess, the superficial properties, the consistency, the oily and/or aqueous deposit, the granularity, formation of a protuberance.

**Table 5:**

| **Time (weeks)** | **Macroscopic and sensory properties of the composition** |
|---|---|
| T0 (After manufacture) | dimensionally stable, ethanol odour, yellow, smooth, shiny composition showing no fluidity |
| T+2 weeks stress test (RT-60°C-RT) | Identical to T0 |
| T+4 weeks at +4°C | Identical to T0 |
| T+4 weeks at RT | Identical to T0 |
| T+4 weeks at +30°C | Identical to T0 |
| T+4 weeks at +40°C | Identical to T0 |
| T+8 weeks at +4°C | Identical to T0 |
| T+8 weeks at RT | Slightly gritty when stored in a tube otherwise identical to T0 |
| T+8 weeks at +30°C | Identical to T0 |
| T+8 weeks at +40°C | Slightly gritty when stored in a tube otherwise identical to T0 |
| T+12 weeks at +4°C | Identical to T0 |
| T+12 weeks at RT | Slightly gritty when stored in a tube otherwise identical to T0 |
| T+12 weeks at +30°C | Identical to T0 |
| T+12 weeks at +40°C | Minimum oil deposits, slightly gritty when stored in a tube otherwise identical to T0 |
| T+26 weeks at +4°C | Identical to T0 |
| T+26 weeks at RT | Slightly gritty when stored in a tube otherwise identical to T0 |
| T+26 weeks at +30°C | Identical to T0 |
| T+26 weeks at +40°C | No visible deposits but gritty when stored in a tube |

### Example 7: Galenic evaluation of the composition 43:

The quality and the stability of the composition 43 were evaluated under the microscope and by a temperature test.

The results of the evaluation under the microscope of the composition 43 (Axioskop, Zeiss) are provided in table 6 herein below:

**Table 6:**

| **Time (weeks)** | **Evaluation with polarizer** | **Evaluation on brightfield** |
|---|---|---|
| T0 (After manufacture) | Absence of crystals | Homogeneous composition, gel structure No presence of droplets |
| T+4 weeks at room temperature (RT) | Absence of crystals | Homogeneous composition, gel structure No presence of droplets |
| T+8 weeks at RT | Absence of crystals | Homogeneous composition, gel structure No presence of droplets |
| T+8 weeks at +4°C | Absence of crystals | Homogeneous composition, gel structure No presence of droplets |
| T+12 weeks at RT | Absence of crystals | Homogeneous composition, gel structure No presence of droplets |
| T+12 weeks at +4°C | Absence of crystals | Homogeneous composition, gel structure No presence of droplets |

The results of the temperature tests of the composition 43 are provided in table 7 herein below. During the temperature tests, sensory and macroscopic parameters were verified, such as in particular the colour, the change in colour, the odour, the shininess, the superficial properties, the consistency, the oily and/or aqueous deposit, the granularity, formation of a protuberance.

**Table 7:**

| **Time (weeks)** | **Macroscopic and sensory properties of the composition** |
|---|---|
| T0 (After manufacture) | ethanol odour, minimal gritty, yellow, shiny, dimensionally stable, composition showing no fluidity |
| T+2 weeks stress test (RT-60°C-RT) | Minimal oil deposits on the surface |
| T+4 weeks at +4°C | Identical to T0 |
| T+4 weeks at RT | Identical to T0 |
| T+4 weeks at +30°C | Identical to T0 |
| T+4 weeks at +40°C | Identical to T0 |
| T+8 weeks at +4°C | Identical to T0 |
| T+8 weeks at RT | Lumpy when stored in a tube otherwise identical to T0 |
| T+8 weeks at +30°C | Identical to T0 |
| T+8 weeks at +40°C | Lumpy when stored in a tube otherwise identical to T0 |
| T+12 weeks at +4°C | Identical to T0 |
| T+12 weeks at RT | Lumpy when stored in a tube otherwise identical to T0 |
| T+12 weeks at +30°C | Identical to T0 |
| T+12 weeks at +40°C | Lumpy when stored in a tube otherwise identical to T0 |

### Example 8: Galenic evaluation of the composition 44:

The quality and the stability of the composition 44 were evaluated under the microscope and by a temperature test.

The results of the evaluation under the microscope of the composition 44 (Axioskop, Zeiss) are provided in table 8 herein below:

**Table 8:**

| **Time (weeks)** | **Evaluation with polarizer** | **Evaluation on brightfield** |
|---|---|---|
| T0 (After manufacture) | Absence of crystals | Homogeneous composition, gel structure Droplets with a size of less than 5µm |
| T+4 weeks at room temperature (RT) | Absence of crystals | Homogeneous composition, gel structure Droplets with a size of less than 10µm |
| T+8 weeks at RT | Absence of crystals | Homogeneous composition, gel structure No presence of droplets |
| T+8 weeks at +4°C | Absence of crystals | Homogeneous composition, gel structure No presence of droplets |
| T+12 weeks at RT | Absence of crystals | Homogeneous composition, gel structure No presence of droplets |
| T+12 weeks at +4°C | Absence of crystals | Homogeneous composition, gel structure No presence of droplets |

The results of the temperature tests of the composition 44 are provided in table 9 herein below. During the temperature tests, sensory and macroscopic parameters were verified, such as in particular the colour, the change in colour, the odour, the shininess, the superficial properties, the consistency, the oily and/or aqueous deposit, the granularity, formation of a protuberance.

**Table 9:**

| **Time (weeks)** | **Macroscopic and sensory properties of the composition** |
|---|---|
| T0 (After manufacture) | Yellow, smooth, shiny, ethanol odour, dimensionally stable composition showing no fluidity and being not gritty |
| T+2 weeks stress test (RT-60°C-RT) | Minimal oil deposits on the surface |
| T+4 weeks at +4°C | Identical to T0 |
| T+4 weeks at RT | Identical to T0 |
| T+4 weeks at +30°C | Identical to T0 |
| T+4 weeks at +40°C | Identical to T0 |
| T+8 weeks at +4°C | Identical to T0 |
| T+8 weeks at RT | Slightly gritty (cottage cheese effect) when stored in a tube otherwise identical to T0 |
| T+8 weeks at +30°C | Identical to T0 |
| T+8 weeks at +40°C | Gritty (cottage cheese effect) when stored in a tube otherwise identical to T0 |
| T+12 weeks at +4°C | Identical to T0 |
| T+12 weeks at RT | Slightly gritty (cottage cheese effect) when stored in a tube otherwise identical to T0 |
| T+12 weeks at +30°C | Identical to T0 |
| T+12 weeks at +40°C | Gritty (cottage cheese effect) when stored in a tube otherwise identical to T0 |

### Example 9: Galenic evaluation of the composition 45:

The quality and the stability of the composition 45 were evaluated under the microscope and by a temperature test.

The results of the evaluation under the microscope of the composition 45 (Axioskop, Zeiss) are provided in table 10 herein below:

**Table 10:**

| **Time (weeks)** | **Evaluation with polarizer** | **Evaluation on brightfield** |
|---|---|---|
| T0 (After manufacture) | Absence of crystals | Homogeneous composition, gel structure No presence of droplets |
| T+4 weeks at room temperature (RT) | Absence of crystals | Homogeneous composition, gel structure No presence of droplets |
| T+8 weeks at RT | Absence of crystals | Homogeneous composition, gel structure No presence of droplets |
| T+8 weeks at +4°C | Absence of crystals | Homogeneous composition, gel structure No presence of droplets |
| T+12 weeks at RT | Absence of crystals | Homogeneous composition, gel structure No presence of droplets |
| T+12 weeks at +4°C | Absence of crystals | Homogeneous composition, gel structure No presence of droplets |

The results of the temperature tests of the composition 43 are provided in table 11 herein below. During the temperature tests, sensory and macroscopic parameters were verified, such as in particular the colour, the change in colour, the odour, the shininess, the superficial properties, the consistency, the oily and/or aqueous deposit, the granularity, formation of a protuberance.

**Table 11:**

| **Time (weeks)** | **Macroscopic and sensory properties of the composition** |
|---|---|
| T0 (After manufacture) | Ethanol odour, yellow, shiny, dimensionally stable, slightly gritty composition showing no fluidity |
| T+2 weeks stress test (RT-60°C-RT) | Minimum water deposits |
| T+4 weeks at +4°C | Identical to T0 |
| T+4 weeks at RT | Identical to T0 |
| T+4 weeks at +30°C | Identical to T0 |
| T+4 weeks at +40°C | Identical to T0 |
| T+8 weeks at +4°C | Identical to T0 |
| T+8 weeks at RT | Slightly gritty (cottage cheese effect) when stored in a tube otherwise identical to T0 |
| T+8 weeks at +30°C | Identical to T0 |
| T+8 weeks at +40°C | Slightly gritty (cottage cheese effect) when stored in a tube otherwise identical to T0 |
| T+12 weeks at +4°C | Identical to T0 |
| T+12 weeks at RT | Slightly gritty (cottage cheese effect) when stored in a tube otherwise identical to T0 |
| T+12 weeks at +30°C | Identical to T0 |
| T+12 weeks at +40°C | Slightly gritty (cottage cheese effect) when stored in a tube otherwise identical to T0 |

### Example 10: Galenic evaluation of the composition 46:

The quality and the stability of the composition 46 were evaluated under the microscope and by a temperature test.

The results of the evaluation under the microscope of the composition 46 (Axioskop, Zeiss) are provided in table 12 herein below:

**Table 12:**

| **Time (weeks)** | **Evaluation with polarizer** | **Evaluation on brightfield** |
|---|---|---|
| T0 (After manufacture) | Absence of crystals | Homogeneous composition, gel structure No presence of droplets |
| T+4 weeks at room temperature (RT) | Absence of crystals | Homogeneous composition, gel structure No presence of droplets |
| T+8 weeks at RT | Absence of crystals | Homogeneous composition, gel structure No presence of droplets |
| T+8 weeks at +4°C | Absence of crystals | Homogeneous composition, gel structure No presence of droplets |
| T+12 weeks at RT | Absence of crystals | Homogeneous composition, gel structure No presence of droplets |
| T+12 weeks at +4°C | Absence of crystals | Homogeneous composition, gel structure No presence of droplets |

The results of the temperature tests of the composition 46 are provided in table 13 herein below. During the temperature tests, sensory and macroscopic parameters were verified, such as in particular the colour, the change in colour, the odour, the shininess, the superficial properties, the consistency, the oily and/or aqueous deposit, the granularity, formation of a protuberance.

**Table 13:**

| **Time (weeks)** | **Macroscopic and sensory properties of the composition** |
|---|---|
| T0 (After manufacture) | Ethanol odour, yellow, shiny, dimensionally stable, gritty composition showing no fluidity |
| T+2 weeks stress test (RT-60°C-RT) | Minimum water deposits |
| T+4 weeks at +4°C | Identical to T0 |
| T+4 weeks at RT | Identical to T0 |
| T+4 weeks at +30°C | Identical to T0 |
| T+4 weeks at +40°C | Identical to T0 |
| T+8 weeks at +4°C | Identical to T0 |
| T+8 weeks at RT | Gritty otherwise identical to T0 |
| T+8 weeks at +30°C | Identical to T0 |
| T+8 weeks at +40°C | Gritty (cottage cheese effect)otherwise identical to T0 |
| T+12 weeks at +4°C | Identical to T0 |
| T+12 weeks at RT | Gritty otherwise identical to T0 |
| T+12 weeks at +30°C | Identical to T0 |
| T+12 weeks at +40°C | Gritty (cottage cheese effect)otherwise identical to T0 |

### Example 11: Galenic evaluation of the composition 47:

The quality and the stability of the composition 47 were evaluated under the microscope and by a temperature test.

The results of the evaluation under the microscope of the composition 47 (Axioskop, Zeiss) are provided in table 14 herein below:

**Table 14:**

| **Time (weeks)** | **Evaluation with polarizer** | **Evaluation on brightfield** |
|---|---|---|
| T0 (After manufacture) | Absence of crystals | Homogeneous composition, gel structure No presence of droplets |
| T+4 weeks at room temperature (RT) | Absence of crystals | Homogeneous composition, gel structure No presence of droplets |
| T+8 weeks at RT | Absence of crystals | Homogeneous composition, gel structure No presence of droplets |
| T+8 weeks at +4°C | Absence of crystals | Homogeneous composition, gel structure No presence of droplets |
| T+12 weeks at RT | Absence of crystals | Homogeneous composition, gel structure No presence of droplets |
| T+12 weeks at +4°C | Absence of crystals | Homogeneous composition, gel structure No presence of droplets |

The results of the temperature tests of the composition 47 are provided in table 15 herein below. During the temperature tests, sensory and macroscopic parameters were verified, such as in particular the colour, the change in colour, the odour, the shininess, the superficial properties, the consistency, the oily and/or aqueous deposit, the granularity, formation of a protuberance.

**Table 15:**

| **Time (weeks)** | **Macroscopic and sensory properties** of the composition |
|---|---|
| T0 (After manufacture) | Ethanol odour, yellow, shiny, dimensionally stable, slightly gritty composition showing no fluidity |
| T+2 weeks stress test (RT-60°C-RT) | oil deposits on the surface |
| T+4 weeks at +4°C | Identical to T0 |
| T+4 weeks at RT | Identical to T0 |
| T+4 weeks at +30°C | Identical to T0 |
| T+4 weeks at +40°C | Identical to T0 |
| T+8 weeks at +4°C | Identical to T0 |
| T+8 weeks at RT | Gritty (cottage cheese effect)when stored in a tube otherwise identical to T0 |
| T+8 weeks at +30°C | Identical to T0 |
| T+8 weeks at +40°C | Gritty (cottage cheese effect)when stored in a tube otherwise identical to T0 |
| T+12 weeks at +4°C | Identical to T0 |
| T+12 weeks at RT | Gritty (cottage cheese effect)when stored in a tube otherwise identical to T0 |
| T+12 weeks at +30°C | Identical to T0 |
| T+12 weeks at +40°C | Minimum oil deposits, gritty (cottage cheese effect)when stored in a tube otherwise identical to T0 |

### Example 12: Galenic evaluation of the composition 48:

The quality and the stability of the composition 48 were evaluated under the microscope and by a temperature test.

The results of the evaluation under the microscope of the composition 48 (Axioskop, Zeiss) are provided in table 16 herein below:

**Table 16:**

| **Time (weeks)** | **Evaluation with polarizer** | **Evaluation on brightfield** |
|---|---|---|
| T0 (After manufacture) | Absence of crystals | Homogeneous composition, gel structure No presence of droplets |
| T+4 weeks at room temperature (RT) | Absence of crystals | Homogeneous composition, gel structure No presence of droplets |
| T+8 weeks at RT | Absence of crystals | Homogeneous composition, gel structure No presence of droplets |
| T+8 weeks at +4°C | Absence of crystals | Homogeneous composition, gel structure No presence of droplets |
| T+12 weeks at RT | Absence of crystals | Homogeneous composition, gel structure No presence of droplets |
| T+12 weeks at +4°C | Absence of crystals | Homogeneous composition, gel structure No presence of droplets |

The results of the temperature tests of the composition 48 are provided in table 17 herein below. During the temperature tests, sensory and macroscopic parameters were verified, such as in particular the colour, the change in colour, the odour, the shininess, the superficial properties, the consistency, the oily and/or aqueous deposit, the granularity, formation of a protuberance.

**Table 17:**

| **Time (weeks)** | **Macroscopic and sensory properties of the composition** |
|---|---|
| T0 (After manufacture) | Yellow, shiny, gritty, ethanol odour, dimensionally stable composition showing no fluidity |
| T+2 weeks stress test (RT-60°C-RT) | Minimal oil deposits on the surface |
| T+4 weeks at +4°C | Identical to T0 |
| T+4 weeks at RT | Identical to T0 |
| T+4 weeks at +30°C | Identical to T0 |
| T+4 weeks at +40°C | Identical to T0 |
| T+8 weeks at +4°C | Identical to T0 |
| T+8 weeks at RT | Gritty (cottage cheese effect)when stored in a tube otherwise identical to T0 |
| T+8 weeks at +30°C | Identical to T0 |
| T+8 weeks at +40°C | Gritty (cottage cheese effect)when stored in a tube otherwise identical to T0 |
| T+12 weeks at +4°C | Identical to T0 |
| T+12 weeks at RT | Gritty (cottage cheese effect)when stored in a tube otherwise identical to T0 |
| T+12 weeks at +30°C | Identical to T0 |
| T+12 weeks at +40°C | Gritty (cottage cheese effect)when stored in a tube otherwise identical to T0 |

### Example 13: Galenic evaluations of the composition 99:

The quality and the stability of the composition 99 were evaluated under the microscope and by a temperature test.

The results of the evaluation under the microscope of the composition 99 (Axioskop, Zeiss) are provided in table 18 herein below:

**Table 18:**

| **Time (weeks)** | **Evaluation with polarizer** | **Evaluation on brightfield** |
|---|---|---|
| T0 (After manufacture) | Absence of crystals | Homogeneous composition No presence of droplets |
| T+4 weeks at room temperature (RT) | Absence of crystals | Homogeneous composition Droplets with a size of less than 10µm |
| T+8 weeks at RT | Absence of crystals | Homogeneous composition No presence of droplets |
| T+8 weeks at +4°C | Absence of crystals | Homogeneous composition No presence of droplets |
| T+12 weeks at RT | Absence of crystals | Homogeneous composition No presence of droplets |
| T+12 weeks at +4°C | Absence of crystals | Homogeneous composition No presence of droplets |
| T+26 weeks at RT | Absence of crystals | Homogeneous composition No presence of droplets |
| T+26 weeks at +4°C | Absence of crystals | Homogeneous composition No presence of droplets |

The results of the temperature tests of the composition 99 are provided in table 19 herein below. During the temperature tests, sensory and macroscopic parameters were verified, such as in particular the colour, the change in colour, the odour, the shininess, the superficial properties, the consistency, the oily and/or aqueous deposit, the granularity, formation of a protuberance.

**Table 19:**

| **Time (weeks)** | **Macroscopic and sensory properties of the composition** |
|---|---|
| T0 (After manufacture) | Yellow, smooth, shiny composition with characteristic odour, not fluid and not gritty |
| T+2 weeks stress test (RT-60°C-RT) | Identical to T0 |
| T+4 weeks at +4°C | Identical to T0 |
| T+4 weeks at RT | Slightly gritty when stored in a tube (due to small air bubbles, no cottage cheese effect) otherwise identical to T0 |
| T+4 weeks at +30°C | Identical to T0 |
| T+4 weeks at +40°C | Slightly gritty when stored in a tube (due to small air bubbles, no cottage cheese effect) otherwise identical to T0 |
| T+8 weeks at +4°C | Identical to T0 |
| T+8 weeks at RT | Slightly gritty when stored in a tube (due to small air bubbles, no cottage cheese effect) otherwise identical to T0 |
| T+8 weeks at +30°C | Identical to T0 |
| T+8 weeks at +40°C | Identical to T0 |
| T+12 weeks at +4°C | Identical to T0 |
| T+12 weeks at RT | Slightly gritty when stored in a tube (due to small air bubbles, no cottage cheese effect) otherwise identical to T0 |
| T+12 weeks at +30°C | Identical to T0 |
| T+12 weeks at +40°C | Light oil deposits on the surface otherwise identical to T0 |
| T+26 weeks at +4°C | Identical to T0 |
| T+26 weeks at RT | Slightly gritty when stored in a tube (due to small air bubbles, no cottage cheese effect) otherwise identical to T0 |
| T+26 weeks at +30°C | Identical to T0 |
| T+26 weeks at +40°C | Light oil deposits on the surface, not gritty, otherwise identical to T0 |

As indicated at example 1, no "cottage cheese effect" was observed for composition 99.

### Example 14: Galenic evaluations of the compositions 166:

The quality and the stability of the composition 166 were evaluated under the microscope and by a temperature test.

The results of the evaluation under the microscope of the composition 166 (Axioskop, Zeiss) are provided in table 20 herein below:

**Table 20:**

| **Time (weeks)** | **Evaluation with polarizer** | **Evaluation on brightfield** |
|---|---|---|
| T0 (After manufacture) | Absence of crystals | Homogeneous composition No presence of droplets |
| T+4 weeks at room temperature (RT) | Absence of crystals | Homogeneous composition Droplets with a size of less than 10µm |
| T+8 weeks at RT | Absence of crystals | Homogeneous composition Droplets with a size of less than 20µm |
| T+8 weeks at +4°C | Absence of crystals | Homogeneous composition Droplets with a size of less than 10µm |
| T+12 weeks at RT | Absence of crystals | Homogeneous composition Droplets with a size of less than 10µm |
| T+12 weeks at +4°C | Absence of crystals | Homogeneous composition Droplets with a size of less than 10µm |
| T+26 weeks at RT | Absence of crystals | Homogeneous composition Droplets with a size of less than 15µm |
| T+26 weeks at +4°C | Crystals with a size of less than 140pm | Homogeneous composition Droplets with a size of less than 10µm |

The results of the temperature tests of the composition 166 are provided in table 21 herein below. During the temperature tests, sensory and macroscopic parameters were verified, such as in particular the colour, the change in colour, the odour, the shininess, the superficial properties, the consistency, the oily and/or aqueous deposit, the granularity, formation of a protuberance.

**Table 21:**

| **Time (weeks)** | **Macroscopic and sensory properties of the composition** |
|---|---|
| T0 (After manufacture) | Yellow, smooth, shiny composition with ethanol odour, not fluid and not gritty |
| T+2 weeks stress test (RT-60°C-RT) | Minimal oil deposits on the surface |
| T+4 weeks at +4°C | Identical to T0 |
| T+4 weeks at RT | Identical to T0 |
| T+4 weeks at +30°C | light oil deposits on the surface |
| T+4 weeks at +40°C | minimal deposition on the surface |
| T+8 weeks at +4°C | Identical to T0 |
| T+8 weeks at RT | Identical to T0 |
| T+8 weeks at +30°C | light oil deposits on the surface |
| T+8 weeks at +40°C | light oil deposits on the surface |
| T+12 weeks at +4°C | Identical to T0 |
| T+12 weeks at RT | Identical to T0 |
| T+12 weeks at +30°C | light oil deposits on the surface |
| T+12 weeks at +40°C | light oil deposits on the surface |
| T+26 weeks at +4°C | Identical to T0 |
| T+26 weeks at RT | light oil deposits on the surface |
| T+26 weeks at +30°C | light oil deposits on the surface |
| T+26 weeks at +40°C | minimal deposition on the surface |

The results shown in tables 20 and 21 hereinabove, show that the composition 166 has qualities that allow it to be formulated as a gel-cream. The results of the galenical evaluation provided in table 20 also show that the composition 166 has a good stability.

As indicated at example 1, no "cottage cheese effect" was observed for composition 166.

To summarize, formulations 41, 99 and 166 containing a lipid phase, two chelating agents and a surfactant do not show the typical cottage cheese effect. Due to the combination of these three excipients, a smooth and stable formulation is obtained. The remaining examples show that leaving out one of these excipients leads to a strong cottage cheese effect again, meaning a cosmetically not acceptable formulation.

## Claims

1. Oil in Water (O/W) emulsion comprising, in a physiologically acceptable medium:
- at least one UV filter;
- an emulsion stabilizing and viscosity controlling agent;
- a gelling agent;
wherein it further comprises at least:
- two chelating agents selected from alphacyclodextrin (Cavamax™ W6 Food) and trisodium Ethylenediamine Disuccinate (Natrlquest™ E30); and
- a surfactant.

2. Emulsion according to one of the preceding claims, wherein the UV filter is chosen from among Bis-Ethylhexyloxyphenol Methoxyphenyl Triazine (BEMT) (Tinosorb™ S), Ethylhexyl Methoxycinnamate, Diethylamino Hydroxybenzoyl Hexyl Benzoate (Uvinul™ A+), Ethyl Hexyl Triazone (Uvinul™ T 150), Methylene Bis-benzotriazolyl Tetramethylbutylphenol (Tinosorb™ M) or a mixture of Titanium Dioxide, C12-C15 Alkyl Benzoate, Polyglyceryl-2 Dipolyhydroxystearate, Silica, Dimethicone (Parsol™ TX50 AB) taken individually or as a mixture.

3. Emulsion according to one of the preceding claims, wherein the emulsion stabilizing and viscosity controlling agent is acrylate and acrylamide polymers.

4. Emulsion according to claim 3, wherein the emulsion stabilizing and viscosity controlling agent is Acrylates/C10-C30 Alkyl Acrylate Crosspolymer (PemulenTM TR2) .

5. Emulsion according to one of the preceding claims, wherein the gelling agent is xanthan gum (XanturalTM 75).

6. Emulsion according to one of the preceding claims, wherein the surfactant is Lauryl Glucoside (PlantacareTM 1200 UP) or Carbopol Ultrez 20.

7. Emulsion according to one of the preceding claims, wherein it further comprises one or several components, taken individually or as a mixture, such as:
- an emollient, such as:
o benzoate esters such as Dipropylene Glycol Dibenzoate (Finsolv® PG-22), Ethylhexyl Benzoate (Finsolv® EB) or C12-15 Alkyl Benzoate;
o Dicaprylyl Carbonate (Cetiol® CC) and
∘ Dibutyl Adipate;
- pH regulators such as Triethanolamine (Trolamine™ EP) and Sodium Hydroxide solution;
- a film-forming agent, such as acetyl tributyl citrate, Antaron™ polymers such as the VP/Hexadecene copolymer (Antaron™ (Ganex) V-216 Polymer), the VP/Eicosene copolymer (Antaron™ (Ganex) V-220/220F polymer and Triacontanyl PVP (Antaron™ (Ganex) WP-660 polymer); waxes such as in particular Cera Alba, Cera candelilla or Cera carnauba; cellulose and derivatives thereof; and chitosan and derivatives thereof;
- skin-conditioning agents such as Dimethicone 5 CS;
- an antioxidant such as dl-alpha-Tocopherol;
- a co-solvent such as ethanol; and
- purified water.

8. Composition according to one of the preceding claims, wherein it comprises the following constituents in the following proportions, expressed as a percentage in weight of the total weight of the composition:
- 30% to 70% purified water;
- 10% to 40% of one or more UV filters which are chosen from among Bis-Ethylhexyloxyphenol Methoxyphenyl Triazine (BEMT) (Tinosorb™ S), Ethylhexyl Methoxycinnamate, Diethylamino Hydroxybenzoyl Hexyl Benzoate (Uvinul™ A+), Ethyl Hexyl Triazone (Uvinul™ T 150), Methylene Bis-benzotriazolyl Tetramethylbutylphenol (Tinosorb™ M) or a mixture of Titanium Dioxide, C12-C15 Alkyl Benzoate, Polyglyceryl-2 Dipolyhydroxystearate, Silica, Dimethicone (Parsol™ TX50 AB) taken individually or as a mixture;
- 0.05% to 0.3% of an emulsion stabilizing and viscosity controlling agent which is Acrylates/C10-30 Alkyl Acrylate Crosspolymer (Pemulen™ TR2);
- 0.05% to 0.3% of a gelling agent which is xanthan gum, (Xantural™ 75)
- 0.05% to 3% of a chelating agent which is alphacyclodextrin (Cavamax™ W6 Food) or trisodium Ethylenediamine Disuccinate (Natrlquest™ E30) or a mixture thereof; and
- 0.01% to 0.3% of a surfactant which is Lauryl Glucoside (Plantacare™ 1200 UP or Carbopol Ultrez 20) .

9. Composition according to claim 8, comprising:
- 4.0% to 5.0% of Bis-Ethylhexyloxyphenol Methoxyphenyl Triazine (BEMT) (Tinosorb™ S);
- 8.0% to 10.0% of Ethylhexyl Methoxycinnamate; and
- 5.0% to 6.0% of Diethylamino Hydroxybenzoyl Hexyl Benzoate (Uvinul™ A+).

10. Composition according to claim 8, comprising:
- 4.0% to 5.0% of Bis-Ethylhexyloxyphenol Methoxyphenyl Triazine (BEMT) (Tinosorb™ S),
- 8.0 to 10.0% of Ethylhexyl Methoxycinnamate,
- 6.0% to 7.5% of Diethylamino Hydroxybenzoyl Hexyl Benzoate (Uvinul™ A+),
- 0.5% to 1.0% of Ethyl Hexyl Triazone (Uvinul™ T 150),
- 2.0% to 3.0% of Methylene Bis-benzotriazolyl Tetramethylbutylphenol (Tinosorb™ M)
- 10.0% to 20.0% of a mixture of Titanium Dioxide, C12-C15 Alkyl Benzoate, Polyglyceryl-2 Dipolyhydroxystearate, Silica, Dimethicone (Parsol™ TX50 AB).

11. Composition according to one of the preceding claims, wherein it does not contain any conventional emulsifier.

12. Composition according to claim 11, wherein it does not contain any Hydrophilic/Lipophilic Balance dependent emulsifier.

13. Composition according to one of the preceding claims, wherein it has the form of a gel-cream.

14. Composition according to one of the preceding claims, wherein it comprises one or several UV filters, at concentrations that make it possible to obtain a protection factor of at least 25.

15. Method of manufacturing a composition according to any of claims 1 to 14, wherein it comprises at least the steps of:
- preparing the oily phase by weighing, melting and/or heating of a first phase comprising at least one UV-filter;
- preparing the aqueous phase by weighing, mixing and homogenising of a second phase comprising at least one emulsion stabilizing and viscosity controlling agent, a gelling agent, chelating agents and a surfactant;
- heating and emulsifying the first phase in the second phase and homogenising;
- recovering a composition according to one of claims 1 to 14.

16. Composition according to one of claims 1 to 14, intended to be used for protecting the skin against UV-A and/or UV-B radiation.

## Patentansprüche

1. Öl-in-Wasser (O/W)-Emulsion, die in einem physiologisch akzeptablen Medium umfasst:
- mindestens einen UV-Filter,
- ein Mittel zur Stabilisierung der Emulsion und zur Kontrolle der Viskosität,
- ein Geliermittel,
wobei sie weiterhin mindestens umfasst:
- zwei Chelatbildner, ausgewählt aus Alphacyclodextrin (Cavamax™ W6 Food) und Trinatriumethylendiamin-disuccinat (Natrlquest™ E30), und
- ein Tensid.

2. Emulsion gemäß dem vorangehenden Ansprüche, wobei der UV-Filter ausgewählt ist aus Bis-Ethylhexyloxyphenol-methoxyphenyl-triazin (BEMT) (Tinosorb™ S), Ethylhexyl-methoxycinnamat, Diethylamino-hydroxybenzoyl-hexylbenzoat (U-vinul™ A+), Ethylhexyl-triazon (Uvinul™ T 150), Methylen-bis-benzotriazolyl-tetramethylbutylphenol (Tinosorb™ M) oder einem Gemisch aus Titandioxid, C12-C15-Alkylbenzoat, Polyglyceryl-2-dipolyhydroxystearat, Kieselsäure, Dimeticon (Parsol™ TX50 AB), einzeln oder als Mischung genommen.

3. Emulsion gemäß einem der vorangehenden Ansprüche, wobei das Emulsionsstabilisierungs- und Viskositätskontrollmittel Acrylat- und Acrylamidpolymere sind.

4. Emulsion gemäß Anspruch 3, wobei das Emulsionsstabilisierungs- und Viskositätskontrollmittel Acrylate/C10-C30-Alkylacrylat-Kreuzpolymer (PemulenTM TR2) ist.

5. Emulsion gemäß einem der vorangehenden Ansprüche, wobei das Geliermittel Xanthan (XanturalTM 75) ist.

6. Emulsion gemäß einem der vorangehenden Ansprüche, wobei das Tensid Laurylglucosid (PlantacareTM 1200 UP) oder Carbopol Ultrez 20 ist.

7. Emulsion gemäß einem der vorangehenden Ansprüche, wobei sie ferner eine oder mehrere Komponenten, einzeln oder als Mischung genommen, umfasst, wie zum Beispiel:
- einen Weichmacher, wie zum Beispiel:
o Benzoatester wie zum Beispiel Dipropylenglykol-dibenzoat (Finsolv® PG-22), Ethylhexylbenzoat (Finsolv® EB) oder C12-15-Alkylbenzoat,
o Dicaprylylcarbonat (Cetiol® CC) und
∘ Dibutyladipat,
- pH-Regulatoren wie zum Beispiel Triethanolamin (Trolamine™ EP) und Natriumhydroxidlösung,
- ein filmbildendes Mittel, wie zum Beispiel Acetyltributylcitrat, Antaron™-Polymere, wie zum Beispiel das VP/Hexadecen-Copolymer (Antaron™ (Ganex) V-216-Polymer), das VP/Eicosen-Copolymer (Antaron™ (Ganex) V-220/220F-Polymer und Triacontanyl-PVP (Antaron™ (Ganex) WP-660-Polymer), Wachse, wie zum Beispiel Cera Alba, Cera candelilla oder Cera carnauba, Cellulose und Derivate davon und Chitosan und Derivate davon,
- Hautpflegestoffe wie zum Beispiel Dimeticon 5 CS,
- ein Antioxidationsmittel wie zum Beispiel dl-alpha-Tocopherol,
- ein Co-Lösungsmittel wie zum Beispiel Ethanol
- gereinigtes Wasser.

8. Zusammensetzung gemäß einem der vorangehenden Ansprüche, wobei sie die folgenden Bestandteile in den folgenden Anteilen, ausgedrückt als Gewichtsprozent des Gesamtgewichts der Zusammensetzung, umfasst:
- 30 % bis 70 % gereinigtes Wasser,
- 10 % bis 40 % eines oder mehrerer UV-Filter, die ausgewählt sind aus Bis-Ethylhexyloxyphenol-methoxyphenyl-triazin (BEMT) (Tinosorb™ S), Ethylhexyl-methoxycinnamat, Diethylamino-hydroxybenzoyl-hexylbenzoat (Uvinul™ A+), Ethylhexyl-triazon (Uvinul™ T 150), Methylen-bis-benzotriazolyl-tetramethylbutylphenol (Tinosorb™ M) oder einem Gemisch aus Titandioxid, C12-C15-Alkylbenzoat, Polyglyceryl-2-dipolyhydroxystearat, Kieselsäure, Dimeticon (Parsol™ TX50 AB), einzeln oder als Mischung genommen,
- 0,05 % bis 0,3 % eines Emulsionsstabilisierungs- und Viskositätskontrollmittels, welches Acrylate/C10-C30-Alkylacrylat-Kreuzpolymer (Pemulen™ TR2) ist,
- 0,05 % bis 0,3 % eines Geliermittels, welches Xanthan (Xantural™ 75) ist,
- 0,05 % bis 0,3 % eines Chelatbildners, bei dem es sich um Alphacyclodextrin (Cavamax™ W6 Food) oder Trinatriumethylendiamin-disuccinat (Natrlquest™ E30) oder ein Gemisch davon handelt,
- 0,01 % bis 0,3 % eines Tensids, das Laurylglucosid (Plantacare™ 1200 UP oder Carbopol Ultrez 20) ist.

9. Zusammensetzung gemäß Anspruch 8, umfassend:
- 4,0 % bis 5,0 % Bis-Ethylhexyloxyphenol-methoxyphenyl-triazin (BEMT) (Tinosorb™ S),
- 8,0 % bis 10,0 % Ethylhexyl-methoxycinnamat,
- 5,0 % bis 6,0 % Diethylamino-hydroxybenzoyl-hexylbenzoat (Uvinul™ A+).

10. Zusammensetzung gemäß Anspruch 8, umfassend:
- 4,0 % bis 5,0 % Bis-Ethylhexyloxyphenol-methoxyphenyl-triazin (BEMT) (Tinosorb™ S),
- 8,0 % bis 10,0 % Ethylhexyl-methoxycinnamat,
- 6,0 % bis 7,5 % Diethylamino-hydroxybenzoyl-hexylbenzoat (Uvinul™ A+),
- 0,5 % bis 1,0 % Ethylhexyl-triazon (Uvinul™ T 150),
- 2,0 % bis 3,0 % Methylen-bis-benzotriazolyl-tetramethylbutylphenol (Tinosorb™ M)
- 10,0 % bis 20,0 % eines Gemisches aus Titandioxid, C12-C15-Alkylbenzoat, Polyglyceryl-2-dipolyhydroxystearat, Kieselsäure, Dimeticon (Parsol™ TX50 AB).

11. Zusammensetzung gemäß einem der vorangehenden Ansprüche, wobei sie keinen herkömmlichen Emulgator enthält.

12. Zusammensetzung gemäß Anspruch 11, wobei sie keinen vom hydrophilen/lipophilen balanceabhängiger Emulgator enthält.

13. Zusammensetzung gemäß einem der vorangehenden Ansprüche, wobei sie die Form einer Gel-Creme hat.

14. Zusammensetzung gemäß einem der vorangehenden Ansprüche, wobei sie einen oder mehrere UV-Filter in Konzentrationen enthält, die es ermöglichen, einen Schutzfaktor von mindestens 25 zu erhalten.

15. Verfahren zur Herstellung einer Zusammensetzung gemäß einem der Ansprüche 1 bis 14, wobei es mindestens die folgenden Schritte umfasst:
- Herstellen der Ölphase durch Wiegen, Schmelzen und/oder Erhitzen einer ersten Phase, die mindestens einen UV-Filter umfasst,
- Herstellen der wässrigen Phase durch Wiegen, Mischen und Homogenisieren einer zweiten Phase, die mindestens ein emulsionsstabilisierendes und viskositätskontrollierendes Mittel, ein Geliermittel, Chelatbildner und ein Tensid umfasst,
- Erhitzen und Emulgieren der ersten Phase in der zweiten Phase und Homogenisieren,
- Rückgewinnen einer Zusammensetzung gemäß einem der Ansprüche 1 bis 14.

16. Zusammensetzung gemäß einem der Ansprüche 1 bis 14, dazu vorgesehen, die Haut gegen UV-A- und/oder UV-B-Strahlung zu schützen.

## Revendications

1. Emulsion huile dans eau (H/E) comprenant dans un milieu physiologiquement acceptable:
- au moins un filtre UV;
- un agent stabilisant d'émulsion et régulateur de viscosité;
- un agent gélifiant;
laquelle comprend en outre au moins:
- deux agents chélatants choisis parmi les alphacyclodextrines (Cavamax™ W6 Food) et trisodium éthylènediamine disuccinate (Natrlquest™ E30); et
- un agent tensioactif.

2. Emulsion selon la revendication précédente dans laquelle le filtre UV est choisi parmi les bis-éthylhéxyloxyphénol méthoxyphényltriazine (BEMT) (Tinosorb™ S), éthylhéxylméthoxycinnamate, diéthylamino hydroxybenzoylhéxylbenzoate (Uvinul™ A+), éthylhéxyltriazone (Uvinul™ T 150), méthylène bis-benzotriazolyl tétraméthylbutylphénol (Tinosorb™ M) ou un mélange de dioxyde de titane, d'alkylbenzoate en C₁₂-C₁₅, de dipolyhydroxystéarate de polyglycéryle-2, de silice, de diméthicone (Parsop™ TX50 AB) pris individuellement ou en mélange.

3. Emulsion selon l'une des revendications précédentes, dans laquelle l'agent stabilisant l'émulsion et régulateur de viscosité consiste en polymères d'acrylate et d'acrylamide.

4. Emulsion selon la revendication 3, dans laquelle l'agent stabilisant l'émulsion et régulateur de viscosité est un polymère réticulé d'acrylates/alkyl-acrylate en C₁₀-C₃₀ (Permulen™ TR2).

5. Emulsion selon l'une des revendications précédentes, dans laquelle l'agent gélifiant est la gomme xanthane (Xantural™ 75).

6. Emulsion selon l'une des revendications précédentes, dans laquelle le tensioactif est le lauryl glucoside (Plantacare™ 1200 UP) ou Carbopol Ultrez 20.

7. Emulsion selon l'une des revendications précédentes, dans laquelle elle comprend en outre un ou plusieurs composants, pris individuellement ou en mélange, tel que:
- un émollient, tel que:
∘ les esters de benzoate tels que le dibenzoate de dipropylèneglycol (Finsolv® PG-22), le benzoate d'éthylhéxyle (Finsolv® EB) ou le benzoate d'alkyle en C₁₂₋₁₅
∘ le carbonate de dicaprylyl (Cetiolt CC) et
∘ dibutyladipate;
- des régulateurs de pH tels que la triéthanolamine (Trolamine™ EP) et la solution d'hydroxyde de sodium;
- un agent filmogène, tel que l'acétyltributylcitrate, les polymères d'Antaron™ tels que le copolymère VP/hexadécène (Antaron™ (Ganex) Polymère V-216), le copolymère VP/éicoséne (Antaron™ (Ganex) polymère V-220/220F et le triacontanyl PVP (Antaron™ (Ganex) polymère WP-660); des cires telles que notamment Cera Alba, Cera candelilla ou Cera carnauba; la cellulose et leurs dérivés; et le chitosane et ses dérivés;
- des agents revitalisants tels que diméthicone 5CS;
- un antioxydant tel que le dl-alpha-tocophérol,
- un co-solvant tel que l'éthanol et
- de l'eau purifiée.

8. Composition selon l'une des revendications précédentes, laquelle comprend les constituants suivants dans les proportions suivantes, exprimés en pourcentage en poids du poids total de la composition:
- 30% à 70% d'eau purifiée;
- 10% à 40% d'un ou plusieurs filtres UV choisis parmi Bis-éthylhéxyloxyphénol méthoxyphényltriazine (BEMT) (Tinosorb™ S), éthylhéxyl méthoxycinnamate, diéthylamino hydroxybenzoyl héxylbenzoate (Uvinul™ A+), éthylhéxyltriazone (Uvinul™ 150), méthylène bis-benzotriazolyl tétraméthylbutylphénol (Tinosorb™ M) ou un mélange de dioxyde de titane, benzoate d'alkyle en C₁₂-C₁₅, dipolyhydroxystéarate de polyglycéryle-2, silice, diméthicone (Parsol™ TX50 AB) pris individuellement ou en mélange;
- 0,05% à 0,3% d'un agent stabilisant l'émulsion et régulateur de viscosité qui consiste en un polymère réticulé d'acrylate/ alkyl acrylate en C₁₀-₃₀ (Pemulen™ TR2);
- 0,05% à 0,3% d'un agent gélifiant qui est la gomme de xanthane (Xantura™ 75)
- 0,05% à 3% d'un agent chélatant qui est l'alphacyclodextrine (Cavamax™ W6 Food) ou le disuccinate d'éthylènediamine trisodique (Natrlquest™ E30) ou un mélange de ceux-ci; et
- 0,01% à 0,3% d'un tensioactif qui est le lauryl glucoside (Plantacare™ 1200 UP ou le Carbopol Ultrez 20).

9. Composition selon la revendication 8, comprenant:
- 4,0% à 5,0% de bis-éthylhéxyloxyphénol méthoxyphényltriazine (BEMT) (Tinosorb™ S);
- 8,0% à 10,0% de méthoxycinnamate d'éthylhéxyl; et
- 5,0% à 6,0% de diéthylamino hydroxybenzoyl benzoate d'héxyle (Uvinul™ A+).

10. Composition selon la revendication 8, comprenant:
- 4,0% à 5,0% de bis-éthylhéxyloxyphénol méthoxyphényl Triazine (BEMT) (Tinosorb™ S),
- 8,0 à 10,0% d'éthylhéxyl méthoxycinnamate,
- 6,0% à 7,5% de diéthylamino hydroxybenzoyl héxylbenzoate (Uvinul™ A+),
- 0,5% à 1,0% d'éthylhéxyltriazone (Uvinul™ T 150),
- 2,0% à 3,0% de méthylène bis-benzotriazolyl tétraméthylbutylphénol (Tinosorb™ M)
- 10,0% à 20,0% d'un mélange de dioxyde de titane, benzoate d'alkyle en C₁₂-C₁₅, polyglycéryl-2 dipolyhydroxystéarate, silice, diméthicone (Parsol™ TX50 AB).

11. Composition selon l'une des revendications précédentes, laquelle ne contient aucun émulsifiantconventionnel.

12. Composition selon la revendication 11, dans laquelle elle ne contient pas d'émulsifiant dépendant de l'équilibre hydrophile/lipophile.

13. Composition selon l'une des revendications précédentes, laquelle se présente sous forme de gel-crème.

14. Composition selon l'une des revendications précédentes, **caractérisée en ce qu'**elle comprend un ou plusieurs filtres UV, en des concentrations permettant d'obtenir un facteur de protection d'au moins 25.

15. Procédé de fabrication d'une composition selon l'une quelconque des revendications 1 à 14, où il comprend au moins les étapes de:
- préparation de la phase huileuse par pesée, fusion et / ou chauffage d'une première phase comprenant au moins un filtre UV;
- préparation de la phase aqueuse par pesée, mélange et homogénéisation d'une seconde phase comprenant au moins un agent stabilisant d'émulsion et régulateur de viscosité, un agent gélifiant, des agents chélatants et un tensioactif;
- chauffage et émulsification de la première phase dans la deuxième phase et homogénéiser;
- récupération d'une composition selon l'une des revendications 1 à 14.

16. Composition selon l'une des revendications 1 à 14, destinée à être utilisée pour protéger la peau des rayons UV-A et/ou UV-B.
